# EUROPEAN PATENT SPECIFICATION

(11) **EP 0 497 917 B1**
(45) Date of publication and mention of the grant of the patent: **19.03.1997**
(21) Application number: 91900553.8
(22) Date of filing: 19.10.1990
(51) Int. Cl.: G11B 31/00

(54) **ENTERTAINMENT APPARATUS**
UNTERHALTUNGSEINRICHTUNG
APPAREIL DE DIVERTISSEMENT

(30) Priority: 26.10.1989 US 426771
(43) Date of publication of application: 12.08.1992
(73) Proprietor: PHILLIPS, Dorothy J., Bakersfield, CA 93307 (US)
(72) Inventor: PHILLIPS, Dorothy J., Bakersfield, CA 93307 (US)
(74) Representative: Baillie, Iain Cameron
(86) International application number: US9006021
(87) International publication number: WO9106953

(56) References cited:
- DE-U- 8 504 773
- FR-A- 1 409 468
- US-A- 69 647
- US-A- 178 276
- US-A- 1 185 987
- US-A- 1 292 105
- US-A- 2 068 590
- US-A- 3 122 130
- US-A- 3 144 992
- US-A- 3 525 862
- US-A- 3 917 283
- US-A- 4 054 286
- US-A- 4 828 527
- US-A- 4 835 556

## Description

### BACKGROUND OF THE INVENTION

### Field of the Invention

The present invention relates to an entertainment apparatus and more particularly to such entertainment apparatus which is suggestive of a theme and which operates in a manner consistent with that theme to provide a soothing atmosphere conducive to relaxation and emotional recovery for use by children, and medical patients of all types, particularly those under treatment for severe or terminal illnesses.

### Description of the Prior Art

There is a wide assortment of devices conventionally available which permit the operator to play recorded music, video recordings such as movies and games, and the like for purposes of entertainment entirely within the control of the operator and dependent upon the operator's choice of audio or video recordings. Similarly, there are a variety of devices which have heretofore been known to project lights or light images in an entertaining fashion substantially or completely within the control of the operator. All such prior art devices permit the operator to choose selections from a wealth of recorded materials.

There has not, however, heretofore been a device operable to assist in the physical and emotional recovery or treatment of seriously ill or terminal patients. Such emotional support is frequently of significant importance in aiding in the recovery of the patient or in providing the support necessary to ease the concerns of a terminal patient. This problem is particularly acute in the case of children suffering from serious or terminal diseases where it is very difficult or impossible to provide emotional comfort to such patients in a manner supportive of their treatment. In the case of children, and particularly small children, these problems are particularly aggravated at night when nurses or other attendants cannot be present at all times to provide the needed comfort.

U.S. Patent No. 4,835,556 describes a cassette player and slide assembly. The device plays a pre-recorded magnetic tape cassette with a voice recording of a performer whose facial image appears on a transparency slide receivable in a holder attached to the player and provided with a window so that, as one hears the performance, one also sees the performer. A light bulb is provided which produces light pulses in synchronism with the reproduced sounds.

Therefore, it has long been known that it would be desirable to have an apparatus operable to provide emotional support to people during these times of stress and particularly to children being treated for serious and terminal diseases during all hours of the day but having particular utility during nighttime hours when attending care is not available.

According to one aspect of the invention there is provided an apparatus operable to interact with a child to provide comfort, the apparatus comprising a housinq having an interior and an external configuration suggestive of a mythological lamp including a base upon which the housing is adapted to rest in upright relation on a surface of support, a spout portion, a handle portion substantially opposite the spout portion and a lid portion substantially midway between the spout portion and the handle portion; a light source mounted in the interior of the housing; a plurality of colored windows mounted on the housing adjacent the lid portion thereof, in spaced relation to each other and so arranged as to suggest that they expose the interior of the housing; an audio player mounted in the interior ot the housing operable to play an audio recording suggestive of a mythological force; a pressure switch operable by the application of rubbing pressure thereto mounted on the housing so as to be engageable by a child externally of the housing; an electrical system operably interconnecting the light source audio player and pressure switch; and a sequencing means mounted in the interior of the housing and operably connected to the electrical system controlling relation therethrough to the light source, the audio player and in controlled relation to the pressure switch so as to be operable by the pressure switch to initiate a predetermined sequence of operation of the light source an audio player whereby a child can apply rubbing pressure to the pressure switch) to illuminate the light source and activate the audio player in accordance with the predetermined sequence of operation to emit light from the interior of the housing through the colored windows and play the audio recording to suggest to the child that a mythological force is present within the housing and has been awakened by rubbing of a mythological lamp.

The apparatus may be particularly well suited to creating an atmosphere by way of a preprogrammed audio message in combination with light transmission suggestive of a theme which is conducive to creating a soothing and relaxing atmosphere, and to affording emotional support to seriously ill and terminal child patients during nighttime hours when nurses and other attendants are not consistently available to provide such support.

The apparatus conveniently incorporates preprogrammed audio and visual messages suggestive of a theme affording the emotional support required by convalescing patients. The apparatus may operate during a first cycle of operation to afford a predetermined audio program in combination with light transmission to suggest a comforting theme and which during a second cycle of operation operates in the manner of a night light consistent with the theme to promote sleep while affording the reassurance of a presence near the patient.

Such an apparatus may possess a simplicity of operation suitable for operation by children of nearly any age while conversely providing a complex program of supportive transmissions suggestive of a strong and comforting presence in the room. The apparatus could be suggestive of a mythological lamp housing a mythological force capable of comforting the patient. It may be of compact form conveniently available for moving to any location and operable without prior adjustment from a conventional electrical power outlet. The apparatus may combine both audio and visual components to create a visual and audio impression fully compatible with the exterior configuration of the apparatus.

In a preferred embodiment of the present invention, there is provided a housing bearing an exterior configuration suggestive of a mythological lamp, mounting an internal audio player in the housing operable to play a preprogrammed audio program, a light source mounted in the housing selectively operable to transmit light through transparent portions of the housing and an electrical system selectively operable to operate the audio player and the light source in a predefined sequence to create audio and visual impressions consistent with a theme created by the exterior configuration of the housing to provide comfort to persons and particularly convalescing patients of young age.

### BRIEF DESCRIPTION OF THE DRAWINGS

Fig. 1 is a side elevation of the entertainment apparatus of the present invention.

Fig. 2 is a top plan view of the entertainment apparatus of Fig. 1.

Fig. 3 is a rear plan view of the apparatus.

Fig. 4 is a front elevation of the apparatus.

Fig. 5 is a longitudinal vertical section taken on line 5-5 in Fig. 2.

Fig. 6 is a schematic diagram of the electrical system of the apparatus of the present invention.

### DESCRIPTION OF THE PREFERRED EMBODIMENT

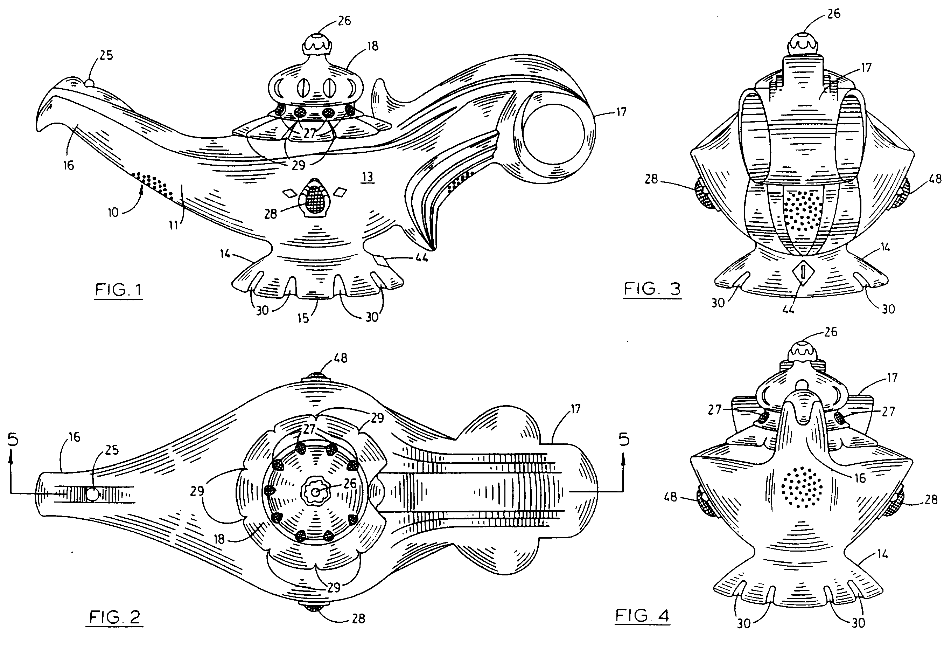

Referring more particularly to the drawings, the apparatus of the present invention is generally indicated by the numeral 10 in Fig. 1. As will subsequently become more clearly apparent, the apparatus of the present invention can be constructed in a wide variety of specific embodiments without departing from the invention. It is the objective in the preferred embodiment of the present invention to create a mood which is soothing while affording a sense of strength which is comforting to the patient. This same objective can be achieved with a wide variety of themes suited to this purpose. However, in the preferred embodiment shown herein, the theme is centered around the suggestion of a mythological lamp which can be operated to transmit a comforting audio message in combination with a sufficient light transmission to soothe the patient. This is achieved in the preferred embodiment by suggesting a lamp housing a power, such as a genie, which not only conveys the preprogrammed message but offers a warm and comforting presence in the patient's room.

Thus, the apparatus 10 has a housing 11 consisting preferably of a molded plastic or ceramic wall 12 having an exterior configuration 13 suggestive or resembling a mythological lamp. As such, the housing has a base 14 with a bottom surface 15. The housing 11 is adapted to rest on its bottom surface on a suitable surface of support such as that of a table or the like. The housing has a spout portion 16 and an opposite handle portion 17. The housing has a plume or lid portion 18.

The spout portion 16 of the housing 11 has a transparent portion on the upper surface thereof constituting a spout light window 25. Similarly, the plume portion 18 of the housing has a transparent portion constituting an upper plume light window 26. A plurality of colored windows 27 are mounted in the plume portion extending in spaced relation thereabout, as can perhaps best be seen in Fig. 2. Similarly, a colored window 28 is provided in the housing substantially centrally thereof above the base 14. At the juncture of the plume portion 18 with the main portion of the housing, the wall 12 has a plurality of translucent portions 29 extending thereabout in spaced relation as can also best be seen in Fig. 2. Similarly, the base has a plurality of translucent portions 30 in the wall 12 extending thereabout. The light windows, colored windows and translucent portions are adapted to emit light from sources within the housing hereinafter to be described in such a manner as to suggest the warm and comforting presence referred to previously. In this regard, of course, the particular arrangement of the lights, coloring thereof, transparency or translucency thereof and the like may be varied as best to suit the mood to be created. However, the colored windows 27 and 28, in the preferred embodiment, have a red color and are cut in such a fashion so as to suggest a ruby or other jewel.

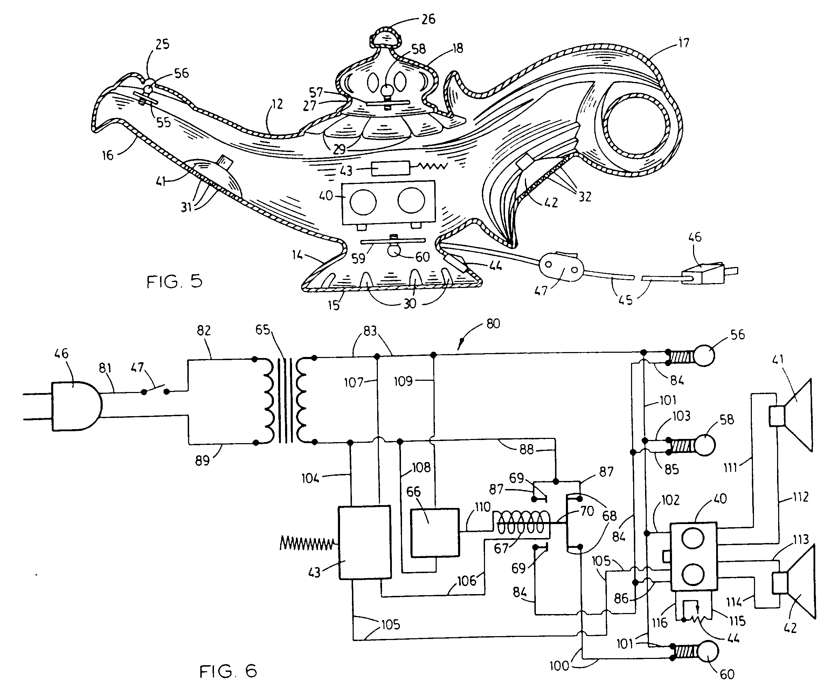

The spout portion 16 of the housing 11 has a plurality of speaker perforations 31 on the underside thereof as best shown in Fig. 5. Similarly, the handle portion 17 has a plurality of speaker perforations 32 on the underside thereof as best shown in Fig. 5.

Referring more particularly to Figs. 5 and 6, an audio tape player 40 is mounted in the interior of the housing 11. The audio tape player is preferably so constructed as to play a single recording suited to the theme and mood adopted for the objectives previously described. A speaker 41 is mounted within the housing 11 in communication with the speaker perforations 31. A speaker 42 is mounted within the housing and in communication with the speaker perforations 32. A pressure switch 43 is mounted within the housing on the back wall as viewed in Fig. 5. A volume switch 44 is mounted on the base 14 communicating with the exterior of the housing, as best shown in Fig. 5. A power cable 45 is extended from the interior of the housing outwardly of the base 14. The power cable has a conventional plug 46 adapted for insertion in a conventional electrical outlet. The power cable mounts a main power switch 47. As can best be seen in Figs. 2, 3 and 4, in keeping with the theme of the embodiment of the invention, the pressure switch 43 mounted internally of the housing 11 communicates with a pressure switch window 48 on the right as viewed in Fig. 3 and on the left as viewed in Fig. 4. The switch window, as shown, appears to be a duplicate of the colored window 28 and may be either transparent or not, as desired. It will be understood, however, that by exerting pressure on the pressure switch window 48, the pressure switch 43 is operated.

As shown in Fig. 5, a mount 55 is mounted in the spout portion 16 and, in turn, mounts a spout light bulb 56 in alignment with the light window 25. A mount 57 is mounted within the housing 11 and, in turn, mounts a plume light bulb 58. A mount 59 is mounted in the housing immediately above the base 14 thereof and, in turn, mounts a base or night light bulb 60.

Referring more particularly to Fig. 6, the apparatus 10 has a step down transformer 65 and a sequencing means, programmable timer/counter or controller 66. The controller may be of any suitable type and is shown herein schematically. The apparatus has a solenoid 67 having a pair of right switch contacts 68 and an opposite pair of left switch contacts 69, as shown in Fig. 6. The solenoid has a plunger 70 operable by operation of the solenoid to move between contacting the right switch contacts and contacting the left switch contacts for purposes hereinafter to be described.

The apparatus 10 has an electrical circuit 80, shown in Fig. 6. The electrical circuit has an electrical conductor 81 interconnecting the plug 46 and the main power switch 47 in the power cable 45. electrical conductor 82 interconnects the main power switch and the transformer 65. Electrical conductor 83 interconnects the transformer and the light bulb 56. Electrical conductor 84 connects the light bulb 56 and the lower switch contact 69 on the left as viewed in Fig. 6 of the solenoid 67. Electrical conductor 85 interconnects the light bulb 58 and the electrical conductor 84. Electrical conductor 86 interconnects the audio tape player 40 and the electrical conductor 84. Electrical conductor 87 interconnects the upper left switch contact 69 and the upper right switch contact 68, as viewed in Fig. 6. Electrical conductor 88 interconnects electrical conductor 87 and the transformer 65. Electrical conductor 89 interconnects the transformer and the plug 46 through the power cable 45.

Electrical conductor 100 interconnects the lowermost right switch contact 68 and the light bulb 60. Electrical conductor 101 interconnects the light bulb 60 and the electrical conductor 83. Electrical conductor 102 interconnects the audio tape player 40 and the electrical conductor 101. Electrical conductor 103 interconnects the light bulb 58 and the electrical conductor 101.

Electrical conductor 104 interconnects the electrical conductor 88 and the pressure switch 43. Electrical conductor 105 interconnects the pressure switch 43 and the audio tape player 40. Electrical conductor 106 interconnects the pressure switch 43 and the solenoid 67. Electrical conductor 107 interconnects the electrical conductor 83 and the pressure switch 43. Electrical conductor 108 interconnects electrical conductor 88 and controller 66. Electrical conductor 109 interconnects the electrical conductor 83 and the controller 66. Electrical conductor 110 interconnects the controller 66 and the solenoid 67.

Electrical conductor 111 interconnects the audio tape player 40 and the speaker 41. Electrical conductor 112 interconnects the speaker 41 and the audio tape player 40. Electrical conductor 113 interconnects the audio tape player 40 and the speaker 42. Electrical conductor 114 interconnects the speaker 42 and the audiotape player 40. Electrical conductor 115 interconnects the audiotape player 40 and the volume control switch 44. Electrical conductor 116 interconnects the volume control switch 44 and the audio tape player 40.

### OPERATION

The operation of the described embodiment of the present invention is believed to be readily apparent and is briefly summarized at this point.

The apparatus 10 is positioned at any suitable location for the purposes heretofore described. For purposes of illustrative convenience, it will be understood that the apparatus is positioned in rested relation on a table adjacent to the bed of a patient of child age. Thus, the bottom surface 15 of the base 14 is rested on the surface of the table adjacent to the bed. In order to place the apparatus in operative condition, the plug 46 is plugged into an available electrical outlet, not shown, and the main power switch 47 is closed to supply power to the transformer 65 and the controller 66. In this condition, the electrical circuit 80 is not activated beyond this supply of power since the pressure switch 43 is disposed in a normally open condition.

If the patient or the attendant wishes to activate the device, the pressure switch window 48 is contacted so as to operate the pressure switch 43. The pressure switch is adjusted such that the operation is activated by rubbing. This action causes the pressure switch to close and thereby complete the circuit through the solenoid 67 and the controller 66.

Depending upon the type and program of the controller 66, such activation preferably causes the controller to supply electrical current to the solenoid which causes the plunger 70 thereof to be drawn to the left as viewed in Fig. 6 to contact the left switch contacts 69. This causes the spout light bulb 56, the plume light bulb 58 and the audio tape player 40 to be activated through electrical conductor 84. Thus, as can best be visualized in Fig. 5, the lights 56, 58 and the tape player are activated so that light emanates through the light window 25, the light window 26, the colored windows 27 and the translucent portions 29. Similarly, the audio tape player plays the recording adapted to provide a soothing and reassuring environment to the patient. The audio signal is transmitted through the speakers 41 and 42 from the apparatus. During this time, the patient or attendant can adjust the volume from the speakers by adjusting the volume switch 44 on the base 14 of the apparatus.

When the audio tape player 40 has run the recording through to the end and the controller 66 has allotted sufficient time for this to occur, the controller 66 operates the solenoid 67 to move the plunger to the right as viewed in Fig. 6 into contact with the switch contact 68. This causes the spout light bulb 56 and the plume light bulb 58 to be deenergized. This also activates the audiotape player 40 through electrical conductor 102 to rewind the tape thereof to a new start position. This also activates the night light 60 thereby causing light to emanate through the colored window 28, and the translucent portions 30 of the base 14 of the apparatus. The apparatus will continue in this cycle of operation until the pressure switch window 48 is again contacted to operate and open the pressure switch 43. This breaks the circuit and de-activates the night light 60 so that the device is completely inoperative until the pressure switch window 48 is again contacted to start the cycle over.

Therefore, the entertainment apparatus of the present invention is operable to provide emotional support to people during times of stress and particularly to children being treated for serious and terminal diseases during all hours of the day but having particular utility during nighttime hours when attending care is not available.

Although the invention has been herein shown and described in what is conceived to be the most practical and preferred embodiment, it is recognized that departures may be made therefrom within the scope of the attached claims.

## Claims

1. An apparatus (10) operable to interact with a child to provide comfort, the apparatus comprising:
a housing (11) having an interior and an external configuration (13) suggestive of a mythological lamp including a base (14) upon which the housing (11) is adapted to rest in upright relation on a surface of support, a spout portion (16), a handle portion (17) substantially opposite the spout portion (16) and a lid portion (18) substantially midway between the spout portion (16) and the handle portion (17);
a light source (56, 58, 60) mounted in the interior of the housing;
a plurality of colored windows (26, 27, 28, 29, 30) mounted on the housing (11) adjacent the lid portion (18) thereof, in spaced relation to each other and so arranged as to suggest that they expose the interior of the housing (11);
an audio player (40) mounted in the interior of the housing (11) operable to play an audio recording suggestive of a mythological force;
a pressure switch (43, 48) operable by the application of rubbing pressure thereto mounted on the housing (11) so as to be engageable by a child externally of the housing (11);
an electrical system (80) operably interconnecting the light source (56, 58, 60), audio player (40) and pressure switch (43, 48); and
a sequencing means (66) mounted in the interior of the housing (11) and operably connected to the electrical system (80) in controlling relation thererhrough to the light source (56, 58, 60) and the audio player (40) and in controlled relation to the pressure switch (43, 48) so as to be operable by the pressure switch (43, 48) to initiate a predetermined sequence of operation of the light source (56, 58, 60) and audio player (40) whereby a child can apply rubbing pressure to the pressure switch (48) to illuminate the light source (56, 58, 60) and activate the audio player (40) in accordance with the predetermined sequence of operation to emit light from the interior of the housing (11) through the colored windows (26, 27, 28, 29, 30) and play the audio recording to suggest to the child that a mythological force is present within the housing (11) and has been awakened by rubbing of a mythological lamp.

2. The apparatus as claimed in Claim 1 further characterized by two speakers (41, 42) mounted in concealed relation in the interior of the housing (11), one speaker (41) beneath the spout portion (16) and one speaker (42) beneath the handle portion (17), in audio communication with the exterior of the housing (11) through a plurality of speaker perforations (31, 32) extending through the housing (11) and operably connected to the audio player (40) through the electrical system (80) whereby sound of the predetermined audio recording is transmitted through the two speakers (41, 42) to the exterior of the housing (11) through the plurality of speaker perforations (31, 32) to suggest that the sound emanates from the mythological force within a mythological lamp.

3. The apparatus as claimed in Claim 1 or 2 further comprising:
a primary switch, operable by the application of rubbing pressure thereto, mounted on the housing so as to be operable by a child externally of the housing;
a secondary switching means mounted in the interior of the housing having at least, a first operative condition and a second operative condition;
wherein the electrical system mounted in the interior of the housing is adapted to be connected to a source of electrical energy, and operably interconnect the source of electrical energy, the primary switch, the secondary switching means, the sequencing means, the audio player, the light source and the speaker to permit a child to apply rubbing pressure to the housing across the primary switch to operate the primary switch to supply electrical energy from the source of electrical enerqy to the sequencing means whereby the sequencing means moves the secondary switching means between the first condition and the second condition in accordance with the predetermined sequence of operations selectively to supply electrical energy from the source to the audio player and the light source for individual operation thereof in accordance with the sequence of operation so as to suggest the existence of a supportive power within the lamp by the playing of the preselected audio recording and the emanation of light from the light source through the colored windows.

## Patentansprüche

1. Vorrichtung (10), die so funktionsfähig ist, daß sie eine Interaktion mit einem Kind vorsieht, um für entsprechende Unterhaltung zu sorgen, wobei die Vorrichtung folgendes umfaßt:
ein Gehäuse (11) mit einer inneren und äußeren Konfiguration (13), die an eine Wunderlampe erinnert, mit einem Sockel (14), auf dem das Gehäuse (11) aufrecht auf einer tragenden Oberfläche ruhen kann, und mit einem Schnauzenabschnitt (16), einem Griffabschnitt (17), der praktisch entgegengesetzt zu dem Schnauzenabschnitt (16) angeordnet ist,und mit einem Deckelabschnitt (18), der sich im wesentlichen in der Mitte zwischen dem Schnauzenabschnitt (16) und dem Griffabschnitt (17) befindet;
eine Lichtquelle (56, 58, 60), die in dem Inneren des Gehäuses angebracht ist;
eine Mehrzahl farbiger Fenster (26, 27, 28, 29, 30), die neben dem Deckelabschnitt (18) des Gehäuses (11) an diesem angebracht sind, und zwar mit Zwischenabständen zueinander, wobei die Fenster so angeordnet sind, daß sie das Innere des Gehäuses (11) offenbaren;
ein Tonwiedergabegerät (40), das in dem Inneren des Gehäuses (11) angebracht ist, wobei das Gerät eine Tonaufzeichnung wiedergeben kann, die an eine mythologische Kraft erinnert;
einen Druckschalter (43, 48), der durch Ausübung eines reibenden Drucks auf den Schalter betätigt werden kann, wobei der Schalter so an dem Gehäuse (11) angebracht ist, daß er durch ein Kind an der Außenseite des Gehäuses (11) betätigt werden kann;
ein elektrisches System (80), das die Lichtquelle (56, 58, 60), das Tonwiedergabegerät (40) und den Druckschalter (43, 48) funktionsfähig miteinander verbindet; und
eine Folgesteuerungseinrichtung (66), die in dem Inneren des Gehäuses (11) angebracht ist, und die funktionsfähig mit dem elektrischen System (80) verbunden ist, wobei die Einrichtung durch das elektrische System steuerungsfähig mit der Lichtquelle (56, 58, 60), dem Tonwiedergabegerät (40) sowie mit dem Druckschalter (43, 48) verbunden ist, so daß die Einrichtung durch den Druckschalter (43, 48) derart betätigt werden kann, daß eine vorbestimmte Funktionsfolge der Lichtquelle (56, 58, 60) und des Tonwiedergabegeräts (40) ausgelöst werden kann, wobei ein Kind einen Reibungsdruck auf den Druckschalter (48) ausüben kann, um die Lichtquelle (56, 58, 60) zum Leuchten zu bringen, und um das Tonwiedergabegerät (40) gemäß der vorbestimmten Funktionsfolge so zu aktivieren, daß aus dem Inneren des Gehäuses (11) durch die farbigen Fenster (26, 27, 28, 29, 30) Licht ausgestrahlt und die Tonaufzeichnung wiedergeben werden, um einem Kind zu suggerieren, daß in dem Gehäuse (11) eine mythologische Kraft vorhanden ist, die durch Reiben der Wunderlampe erweckt wird.

2. Vorrichtung nach Anspruch 1, ferner gekennzeichnet durch zwei Lautsprecher (41, 42), die verdeckt in dem Inneren des Gehäuses (11) angebracht sind, wobei sich ein Lautsprecher (41) unter dem Schnauzenabschnitt (16) befindet, während der andere Lautsprecher (42) hinter dem Griffabschnitt (17) angeordnet ist, wobei die Lautsprecher durch eine Mehrzahl von Lautsprecheröffnungen (31, 32) eine Tonübertragungsverbindung aus dem Gehäuse hinaus aufweisen, wobei sich die Lautsprecheröffnungen durch das Gehäuse (11) erstrecken und über das elektrische System (80) funktionsfähig mit dem Tonwiedergabegerät (40) verbunden sind, wobei der Ton der vorbestimmten Tonaufzeichnung durch die Lautsprecher (41, 42) durch die Mehrzahl von Lautsprecheröffnungen (31, 32) aus dem Gehäuse (11) hinaus übertragen werden kann, so daß der Eindruck erweckt wird, daß der Ton von der mythologischen Kraft in der mythologischen Lampe stammt.

3. Vorrichtung nach Anspruch 1 oder 2, wobei die Vorrichtung ferner folgendes umfaßt:
einen Hauptschalter, der durch Ausübung einer reibenden Kraft auf den Schalter betätigt werden kann, wobei der Schalter so an dem Gehäuse angebracht ist, daß er von einem Kind von außerhalb des Gehäuses aktiviert werden kann;
eine in dem Inneren des Gehäuses angebrachte Nebenschalteinrichtung mit mindestens einem ersten Betriebszustand und einem zweiten Betriebszustand;
wobei das in dem Inneren des Gehäuses angebrachte elektrische System mit einer Quelle elektrischer Energie vebunden werden kann, und wobei es die Quelle elektrischer Energie, den Hauptschalter, die Nebenschalteinrichtung, die Folgesteuerungseinrichtung, das Tonwiedergabegerät, die Lichtquelle und die Lautsprecher funktionsfähig miteinander verbinden kann, so daß ein Kind über den Hauptschalter einen Reibungsdruck auf das Gehäuse ausüben kann, um den Hauptschalter zu betätigen, so daß der Hauptschalter so aktiviert wird, daß von der Quelle elektrischer Energie elektrische Energie der Folgesteuerungseinrichtung zugeführt wird, wobei die Folgesteuerungseinrichtung die Nebenschalteinrichtung gemäß der vorbestimmten Funktionsfolge zwischen dem ersten Zustand und dem zweiten Zustand bewegt, um wahlweise elektrische Energie von der Quelle dem Tonwiedergabegerät und der Lichtquelle zuzuführen, um diese gemäß der Funktionsfolge einzeln zu betätigen, um durch das Abspielen der vorausgewählten Tonaufzeichnung und durch das Austreten von Licht aus der Lichtquelle durch die farbigen Fenster die Existenz einer unterstützenden Kraft in der Lampe zu suggerieren.

## Revendications

1. Appareil (10) fonctionnant en interaction avec un enfant pour créer une distraction, l'appareil comprenant ;
un corps (11), ayant un espace intérieur et une configuration extérieure (13) suggérant une lampe magique, qui comporte une base (14) sur laquelle le corps (11) peut reposer en position debout sur une surface de support, un bec (16), une poignée (17) sensiblement à l'opposé du bec (16), et un couvercle (18) situé sensiblement à mi-distance entre le bec (16) et la poignée (17) ;
une source de lumière (56,58,60) montée à l'intérieur du corps ;
une pluralité de fenêtres colorées (26,27,28,29, 30) montées sur le corps (11) près de son couvercle (18), à distance les unes des autres et agencées de façon à suggérer qu'elles découvrent l'intérieur du corps (11) ;
un électrophone (40) monté à l'intérieur du corps (11) et fonctionnant pour reproduire un enregistrement sonore suggérant une force magique ;
un interrupteur à pression (43,48),fonctionnant par application d'une pression de frottement sur ledit interrupteur, monté sur le corps (11) de façon à pouvoir être touché par un enfant à l'extérieur du corps (11) ;
un système électrique (80) interconnectant fonctionnellement la source de lumière (56,58,60), l'électrophone (40) et l'interrupteur à pression (43,48) ; et
un moyen de séquencement (66) monté à l'intérieur du corps (11) et fonctionnellement connecté au système électrique (80) en relation de commande, par son intermédiaire, avec la source de lumière (56,58,60) et avec l'électrophone (40) et en relation commandée avec l'interrupteur à pression (43,48) afin d'être actionnable par l'interrupteur à pression (43,48) pour démarrer une séquence prédéterminée de fonctionnement de la source de lumière (56,58,60) et de l'électrophone (40), de sorte qu'un enfant peut appliquer une pression de frottement à l'interrupteur à pression (48) pour allumer la source de lumière (56,58,60) et activer l'électrophone (40) conformément à la séquence de fonctionnement prédéterminée, afin d'émettre une lumière venant de l'intérieur du corps (11) à travers les fenêtres colorées (26,27,28,29,30) et de jouer l'enregistrement sonore de manière à suggérer à l'enfant qu'une force magique est présente à l'intérieur du corps (11) et a été réveillée par frottement d'une lampe magique.

2. Appareil suivant la revendication 1, caractérisé en outre en ce qu'il comprend deux haut-parleurs (41,42) montés de façon dissimulée à l'intérieur du corps (11), un haut-parleur (41) étant placé sous le bec (16) et un haut-parleur (42) étant placé sous la poignée (17), en communication acoustique avec l'extérieur du corps (11) par l'intermédiaire d'une pluralité de perforations de haut-parleur(31,32) qui traversent le corps (11), et fonctionnellement connectés à l'électrophone (40) par le système électrique (80), de sorte que le son de l'enregistrement sonore prédéterminé est transmis par l'intermédiaire des deux haut-parleurs (41,42) à l'extérieur du corps (11) à travers la pluralité de perforations de haut-parleur (31 ,32) afin de suggérer que le son émane de la force magique présente à l'intérieur d'une lampe magique.

3. Appareil suivant la revendication 1 ou 2, comprenant en outre :
un interrupteur principal, manoeuvrable par l'application d'une pression de frottement audit interrupteur, monté sur le corps de façon à être actionnable par un enfant à l'extérieur du corps ;
un moyen de commutation secondaire, monté à l'intérieur du corps, ayant au moins un premier état de fonctionnement et un deuxième état de fonctionnement ;
dans lequel le système électrique monté à l'intérieur du corps est prévu pour être connecté à une source d'énergie électrique et pour interconnecter fonctionnellement la source d'énergie électrique, l'interrupteur principal, le moyen de commutation secondaire, le moyen de séquencement, l'électrophone, la source de lumière et les haut-parleurs pour permettre à un enfant d'appliquer une pression de frottement au corps, sur l'interrupteur principal, de manière à manoeuvrer l'interrupteur principal afin de fournir une énergie électrique de la source d'énergie électrique au moyen de séquencement, de sorte que le moyen de séquencement déplace le moyen de commutation secondaire entre le premier état et le deuxième état conformément à la séquence prédéterminée d'opérations sélectivement pour fournir l'énergie électrique de la source à l'électrophone et à la source de lumière afin qu'ils fonctionnent individuellement conformément à la séquence d'opérations de manière à suggérer l'existence d'une force amie à l'intérieur de la lampe par la reproduction de l'enregistrement sonore prédéterminé et l'émission de lumière par la source de lumière à travers les fenêtres colorées.
